# EUROPEAN PATENT SPECIFICATION

(11) **EP 3 720 899 B1**
(45) Date of publication and mention of the grant of the patent: **31.05.2023**
(21) Application number: 18833135.9
(22) Date of filing: 06.12.2018
(51) Int. Cl.: C08K 5/00, C08K 3/013

(54) **CURABLE FLUORINATED POLYMER COMPOSITIONS**
HÄRTBARE FLUORIERTE POLYMERZUSAMMENSETZUNGEN
COMPOSITIONS DURCISSABLES DE POLYMÈRE FLUORÉ

(30) Priority: 08.12.2017 US 201762596309 P; 13.12.2017 US 201762598019 P
(43) Date of publication of application: 14.10.2020
(73) Proprietor: 3M Innovative Properties Company, Saint Paul, Minnesota 55133-3427 (US)
(72) Inventor: GUERRA, Miguel A., Saint Paul, Minnesota 55133-3427 (US); FUKUSHI, Tatsuo, Saint Paul, Minnesota 55133-3427 (US); MITCHELL, Michael H., Saint Paul, Minnesota 55133-3427 (US)
(74) Representative: Mathys & Squire
(86) International application number: PCT/US2018/064202
(87) International publication number: WO 2019/113286

(56) References cited:
- WO-A1-92/12199
- WO-A1-2007/075261
- WO-A1-2017/011379
- WO-A2-00/06533
- US-A1- 2012 202 950

## Description

### TECHNICAL FIELD

Curing of compositions comprising a fluorinated elastomeric gum with a novel curing system are discussed.

### SUMMARY

There is a desire to identify a novel curing system for fluorinated elastomeric gums.

In one aspect, a composition is described. The composition comprising: (a) a fluorinated elastomeric gum comprising at least 0.05 by weight of a cure site, wherein the cure site comprises at least one of bromine, iodine, nitrile, or combinations thereof; and (b) a co-agent of Formula I or a salt thereof, wherein Formula I is:

(CH₂=CHCH₂)₂-L¹-Rf-L²-(CH₂CH=CH₂)ₙ(H)₂₋ₙ

wherein L¹ is -NSO₂- or -NCO-; L² is -SO₂N- or -CON-; n is 1 or 2; and Rf is a divalent perfluorinated carbon group, optionally comprising at least one ether linkage.

In another aspect, a method of curing a fluorinated elastomeric gum is described, the method comprising:
obtaining a fluorinated elastomeric gum comprising cure sites, wherein the cure site comprises at least one of bromine, iodine, nitrile, or combinations thereof;
curing the fluorinated elastomeric gum with a peroxide and a co-agent according to Formula I or a salt thereof, wherein Formula I is: (CH₂=CHCH₂)₂-L¹-Rf-L²-(CH₂CH=CH₂)ₙ(H)₂₋ₙ
wherein L¹ is -NSO₂- or -NCO-; L² is -SO₂N- or -CON-; n is 1 or 2; and Rf is a divalent perfluorinated carbon group, optionally comprising at least one ether linkage.

The above summary is not intended to describe each embodiment. The details of one or more embodiments of the invention are also set forth in the description below. Other features, objects, and advantages will be apparent from the description and from the claims.

Patent application WO2007075261 discloses a composition comprising a fluorocarbon polymer, a radical initiator, and a first curing co-agent. The first curing co-agent comprises at least one silicon-containing group selected from a hydrocarbyl silane and a hydrocarbyl silazane. The first curing co-agent is substantially free of siloxane groups and comprises at least one polymerizable ethylenically unsaturated group.

Patent application WO9212199 discloses fluorocarbon elastomer gum compounded with fluorinated ether compositions, the resulting compounded gum is shaped and heated to form a cured shaped article such as automotive fuel line hose or O-ring.

### DETAILED DESCRIPTION

As used herein, the term
"a", "an", and "the" are used interchangeably and mean one or more;
"and/or" is used to indicate one or both stated cases may occur, for example A and/or B includes, (A and B) and (A or B);
"backbone" refers to the main continuous chain of the polymer;
"crosslinking" refers to connecting two pre-formed polymer chains using chemical bonds or chemical groups;
"cure site" refers to functional groups, which may participate in crosslinking;
"interpolymerized" refers to monomers that are polymerized together to form a polymer backbone;
"monomer" is a molecule which can undergo polymerization which then form part of the essential structure of a polymer;
"perfluorinated" means a group or a compound derived from a hydrocarbon wherein all hydrogen atoms have been replaced by fluorine atoms. A perfluorinated compound may however still contain other atoms than fluorine and carbon atoms, like oxygen atoms, chlorine atoms, bromine atoms and iodine atoms; and
"polymer" refers to a macrostructure having a number average molecular weight (Mn) of at least 50,000 dalton, at least 100,000 dalton, at least 300,000 dalton, at least 500,000 dalton, at least, 750,000 dalton, at least 1,000,000 dalton, or even at least 1,500,000 dalton and not such a high molecular weight as to cause premature gelling of the polymer.

Also herein, recitation of ranges by endpoints includes all numbers subsumed within that range (e.g., 1 to 10 includes 1.4, 1.9, 2.33, 5.75, 9.98).

Also herein, recitation of "at least one" includes all numbers of one and greater (e.g., at least 2, at least 4, at least 6, at least 8, at least 10, at least 25, at least 50, at least 100).

As used herein, the phrase "comprising at least one of" followed by a list refers to comprising any one of the items in the list and any combination of two or more items in the list. The phrase "at least one of" followed by a list refers to any one of the items in the list or any combination of two or more items in the list.

Fluoroelastomers are normally crosslinked when formed into their final part shapes. It is desirable for the crosslinks to have at least as much chemical and thermal stability as the fluoroelastomers themselves. One method of forming crosslinks is via a free radical mechanism initiated by peroxides.

Co-agents are used in peroxide cured fluoropolymers to form the crosslinks between fluoroelastomer polymer chains and may also improve the cure efficiency. One of the most common co-agents used in free radical curing of fluoroelastomers is triallyl isocyanurate (TAIC). Although TAIC provides good mechanical properties at an affordable price point, it does have some drawbacks. Namely, TAIC is known to homopolymerize, can have compatibility issues with some fluoropolymers (especially perfluorinated monomers), can impact processing and/or performance (e.g., causing mold fouling, and/or reducing the high temperature thermal resistance of the fluoroelastomer).

In the present disclosure, it has been found that a fluorinated elastomeric gum can be peroxide cured using the co-agents of Formula I disclosed herein. Use of the Formula I co-agent or salt thereof may provide more compatibility with the fluoropolymer as it comprises a fluorinated segment; may have a reduced tendency to homopolymerize; and/or may impart improved performance properties to the fluoroelastomer.

Co-Agent

The co-agent of the present disclosure is a compound according to Formula I:

(CH₂=CHCH₂)₂-L¹-Rf-L²-(CH₂CH=CH₂)ₙ(H)₂₋ₙ (I)

wherein L¹ is -NSO₂- or -NCO-; L² is -SO₂N- or -CON-; n is 1 or 2; and Rf is a divalent perfluorinated carbon group, optionally comprising at least one ether linkage.

The -NSO₂- and -SO₂N- groups are sulfonamide groups, having a structure of 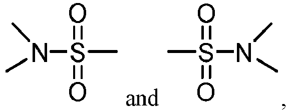 respectively.

The -NCO- and -CON- groups are carboxamide groups, having a structure of 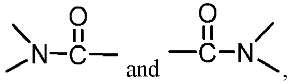 respectively.

Rf represents a divalent group comprising fluorinated carbons and optionally at least one ether linkage. In one embodiment, Rf is linear. In one embodiment, Rf comprises branching. In another embodiment, Rf comprises a non-aromatic cyclic group. In one embodiment, Rf is a perfluorinated alkylene group comprising 1, 2, 3, 4, 5, 6, 7, 8, 9, 10, 11, 12, 13, 14, 15, 16, 17, 18, 19, or even 20 carbon atoms. In another embodiment, Rf is a divalent perfluoro carbon group, comprising 1, 2, 3, 4, 5, 6, 7, 8, 9, 10, 11, 12, 13, 14, 15, 16, 17, 18, 19, or even 20 carbon atoms, and at least one ether linkage.

When n=1, the co-agent of Formula I may be in a salt form such as

(CH₂=CHCH₂)₂-L¹-Rf-L²-(CH₂CH=CH₂)(M_{1/x}) (II)

wherein L¹, L², and Rf are as defined above and M is a salt forming cation with a valence x, wherein x is 1, 2, or 3. Such salt forming cations include, for example, NH₄⁺, H₃O⁺, Na⁺, Li⁺, Cs⁺, Ca⁺², K⁺, Mg⁺², Zn⁺², and Cu⁺², and/or an organic cation including, but not limited to, N(CH₃)₄⁺, NH₂(CH₃)₂⁺, N(CH₂CH₃)₄⁺, NH(CH₂CH₃)₃⁺, NH(CH₃)₃⁺, ((CH₃CH₂CH₂CH₂)₄)P⁺, and combinations thereof.

Exemplary co-agents of Formula I include:

(CH₂=CHCH₂)₂- NSO₂-(CF₂)ₘ-SO₂N-(CH₂CH=CH₂)₂;

(CH₂=CHCH₂)₂- NSO₂-(CF₂)ₘ-CON-(CH₂CH=CH₂)₂;

(CH₂=CHCH₂)₂-NOC-(CF₂)ₘ-CON-(CH₂CH=CH₂)₂;

(CH₂=CHCH₂)₂- NSO₂-(CF₂)ₘ-CON-(CH₂CH=CH₂)H

and salts thereof;

(CH₂=CHCH₂)H- NSO₂-(CF₂)ₘ-CON-(CH₂CH=CH₂)₂

and salts thereof; and

(CH₂=CHCH₂)₂-NSO₂-(CF₂)ₚ-O-(CF₂)_{q}-O-(CF₂)ᵣ₋SO₂N-(CH₂CH=CH₂)₂

wherein m is an integer from 1 to 10; and p, q, and r are independently selected from an integer of 1 to 6.

More specifically, exemplary co-agents of Formula I include

CH₂=CHCH₂)₂NSO₂C₃F₆SO₂N(CH₂CH=CH₂)₂; (CH₂=CHCH₂)₂NSO₂C₄F₈SO₂N(CH₂CH=CH₂)₂;

(CH₂=CHCH₂)₂NSO₂ (cyclic-C₆F₁₀)SO₂N(CH₂CH=CH₂)₂;

(CH₂=CHCH₂)₂NSO₂C₂F₄OC₂F₄SO₂N(CH₂CH=CH₂)₂;

(CH₂=CHCH₂)₂NCOC₄F₈CON(CH₂CH=CH₂)₂;

(CH₂=CHCH₂)₂NCOCF₂OC₂F₄OCF₂CON(CH₂CH=CH₂)₂;

(CH₂=CHCH₂)₂NCOCF₂OC₂F₄OC₂F₄OCF₂CON(CH₂CH=CH₂)₂;

(CH₂=CHCH₂)₂NCOC₃F₆SO₂N(CH₂CH=CH₂)₂;

(CH₂=CHCH₂)₂NCO(cyclic-C₆F₁₀)CON(CH₂CH=CH₂)₂;

CH₂=CHCH₂NHSO₂C₃F₆CON(CH₂CH=CH₂)₂

and salts thereof; and

(CH₂=CHCH₂)₂NSO₂C₃F₆CONHCH₂CH=CH₂

and salts thereof.

In one embodiment, the co-agents according to Formula I have a molecular weight of greater than 350, 400, 450, or even 500 grams/mole; and less than 1000, 1250, 1500, or even 2000 grams/mole.

Co-agents according to Formula I may be formed by methods known in the art. For example, a fluorinated disulfonyl fluoride or fluorinated dicarboxylic acid may be reacted with diallyl amine to form a fluorinated disulfonamide or dicarboxamide tetraallyl. Solvent can be use in the case of fluorinated dicarboxylic acids to control the reaction temperature with diallyl amine. Fluorinated disulfonyl fluorides may be reacted neat with diallyl amine at elevated temperature to get the reaction to go to completion. The final co-agent can be isolated by vacuum distillation.

### Fluorinated Elastomeric Gum

As used herein the phrase "fluorinated elastomeric gum" refers to a fluoropolymer that can be processed as a traditional elastomer. To be processed as a traditional elastomer means that the fluoropolymer that can be processed with a two-roll mill or an internal mixer. Mill blending is a process that rubber manufacturers use to combine the polymer gum with the requisite curing agents and/or additives. In order to be mill blended, the fluorinated elastomeric gum must have a sufficient modulus. In other words, not too soft that it sticks to the mill, and not too stiff that it cannot be banded onto mill. In one embodiment, the fluorinated elastomeric gum of the present disclosure has a modulus of at least 0.1, 0.3, or even 0.5 MPa (megaPascals); and at most 2.5, 2.2, or even 2.0 MPa at 100°C as measured at a strain of 1% and a frequency of 1 Hz (Hertz).

The fluorinated elastomeric gum may be perfluorinated or partially fluorinated. As disclosed herein, in a perfluorinated polymer, the carbon-hydrogen bonds along the backbone of the polymer are all replaced with carbon-fluorine bonds and optionally some carbon-chlorine bonds. It is noted that the backbone of the polymer excludes the sites of initiation and termination of the polymer. As disclosed herein, in a partially fluorinated polymer, the polymer comprises at least one carbon-hydrogen bond and at least one carbon-fluorine bond on the backbone of the polymer excluding the sites of initiation and termination of the polymer. In one embodiment, the fluorinated polymer is highly fluorinated, wherein at least 50, 60, 70, 80, or even 85% of the polymer backbone comprises C-F bonds and at most 90, 95, or even 99%.

In one embodiment, the fluorinated elastomeric gum may be derived from one or more fluorinated monomer(s) such as tetrafluoroethylene (TFE), vinyl fluoride (VF), vinylidene fluoride (VDF), hexafluoropropylene (HFP), pentafluoropropylene, trifluoroethylene, trifluorochloroethylene (CTFE), perfluorovinyl ethers, perfluoroallyl ethers, and combinations thereof.

In one embodiment, perfluorovinyl ethers are of the Formula III

CF₂=CFO(R_{f'}O)ₘR_{f} (III)

where R_{f"} is a linear or branched perfluoroalkylene radical groups comprising 2, 3, 4, 5, or 6 carbon atoms, m is an integer selected from 0, 1, 2, 3, 4, 5, 6, 7, 8, 9, and 10, and R_{f} is a perfluoroalkyl group comprising 1, 2, 3, 4, 5, or 6 carbon atoms. Exemplary perfluorovinyl ether monomers include: perfluoro (methyl vinyl) ether (PMVE), perfluoro (ethyl vinyl) ether (PEVE), perfluoro (n-propyl vinyl) ether (PPVE-1), perfluoro-2-propoxypropylvinyl ether (PPVE-2), perfluoro-3-methoxy-n-propylvinyl ether, perfluoro-2-methoxy-ethylvinyl ether, perfluoro-methoxy-methylvinylether (CF₃-O-CF₂-O-CF=CF₂), and CF₃-(CF₂)₂-O-CF(CF₃)-CF₂-O-CF(CF₃)-CF₂-O-CF=CF₂, and combinations thereof.

In one embodiment, perfluoroallyl ethers are of the Formula IV

CF₂=CFCF₂O(R_{f"}O)ₙ(R_{f'}O)ₘR_{f} (IV)

where R_{f"} and R_{f'} are independently linear or branched perfluoroalkylene radical groups comprising 2, 3, 4, 5, or 6 carbon atoms, m and n are independently an integer selected from 0, 1, 2, 3, 4, 5, 6, 7, 8, 9, and 10, and R_{f} is a perfluoroalkyl group comprising 1, 2, 3, 4, 5, or 6 carbon atoms. Exemplary perfluoroallyl ether monomers include: perfluoro (ethyl allyl) ether, perfluoro (n-propyl allyl) ether, perfluoro-2-propoxypropyl allyl ether, perfluoro-3-methoxy-n-propylallyl ether, perfluoro-2-methoxy-ethyl allyl ether, perfluoro-methoxy-methyl allyl ether, and CF₃-(CF₂)₂-O-CF(CF₃)-CF₂-O-CF(CF₃)-CF₂-O-CF₂CF=CF₂, and combinations thereof.

It is known by those of skill in the art to modify the fluorinated elastomeric gum during the polymer formation by the addition of small amounts of other copolymerizable monomers, which may or may not contain fluorine substitution, e.g. ethylene, propylene, butylene. Use of these additional monomers (i.e., comonomers) is within the scope of the present disclosure. Generally, these additional monomers would be used at less than 25 mole percent of the fluoropolymer, preferably less than 10 mole percent, and even less than 3 mole percent.

In one embodiment, the fluorinated elastomeric gum is a random copolymer, which is amorphous, meaning that there is an absence of long-range order (i.e., in long-range order the arrangement and orientation of the macromolecules beyond their nearest neighbors is understood). An amorphous fluoropolymer has no detectable crystalline character by DSC (differential scanning calorimetry), meaning that if studied under DSC, the fluoropolymer would have no melting point or melt transitions with an enthalpy more than 0.002, 0.01, 0.1, or even 1 Joule/g from the second heat of a heat/cool/heat cycle, when tested using a DSC thermogram with a first heat cycle starting at -85°C and ramped at 10 °C/min to 350°C, cooling to -85°C at a rate of 10°C/min and a second heat cycle starting from -85°C and ramped at 10 °C/min to 350°C. Exemplary amorphous random copolymers may include: copolymers comprising TFE and perfluorinated vinyl ethers monomeric units (such as copolymers comprising TFE and PMVE, and copolymers comprising TFE and PEVE); copolymers comprising TFE and perfluorinated allyl ethers monomeric units; copolymers comprising TFE and propylene monomeric units; copolymers comprising TFE, propylene, and VDF monomeric units; copolymers comprising VDF and HFP monomeric units; copolymers comprising TFE, VDF, and HFP monomeric units; copolymers comprising TFE and ethyl vinyl ether (EVE) monomeric units; copolymers comprising TFE and butyl vinyl ether (BVE) monomeric units; copolymers comprising TFE, EVE, and BVE monomeric units; copolymers comprising VDF and perfluorinated vinyl ethers monomeric units (such as copolymers comprising VDF and CF₂=CFOC₃F₇) monomeric units; an ethylene and HFP monomeric units; copolymers comprising CTFE and VDF monomeric units; copolymers comprising TFE and VDF monomeric units; copolymers comprising TFE, VDF and perfluorinated vinyl ethers monomeric units (such as copolymers comprising TFE, VDF, and PMVE) monomeric units; copolymers comprising VDF, TFE, and propylene monomeric units; copolymers comprising TFE, VDF, PMVE, and ethylene monomeric units; copolymers comprising TFE, VDF, and perfluorinated vinyl ethers monomeric units (such as copolymers comprising TFE, VDF, and CF₂=CFO(CF₂)₃OCF₃) monomeric units; and combinations thereof. In one embodiment, the fluorinated polymer is not a copolymer comprising VDF and HFP monomeric units.

In one embodiment, the fluorinated elastomeric gum is a block copolymer in which chemically different blocks or sequences are covalently bonded to each other, wherein the blocks have different chemical compositions and/or different glass transition temperatures. In one embodiment, the block copolymer comprises a first block, A block, which is a semi-crystalline segment. If studied under a differential scanning calorimetry (DSC), this block would have at least one melting point temperature (Tₘ) of greater than 70°C and a measurable enthalpy, for example, greater than 0 J/g (Joules/gram). The second block, or B block, is an amorphous segment, meaning that there is an absence of long-range order (i.e., in long-range order the arrangement and orientation of the macromolecules beyond their nearest neighbors is understood). The amorphous segment has no detectable crystalline character by DSC. If studied under DSC, the B block would have no melting point or melt transitions with an enthalpy more than 2 milliJoules/g by DSC. In one embodiment, the A block is copolymer derived from at least the following monomers: tetrafluoroethylene (TFE), hexafluoropropylene (HFP), and vinylidene fluoride (VDF). In one embodiment, the A block comprises 30-85 wt (weight) % TFE; 5-40 wt % HFP; and 5-55 wt % VDF; 30-75 wt % TFE; 5-35 wt % HFP; and 5-50 wt % VDF; or even 40-70 wt % TFE; 10-30 wt % HFP; and 10-45 wt % VDF. In one embodiment, the B block is a copolymer derived from at least the following monomers: hexafluoropropylene (HFP), and vinylidene fluoride (VDF). In one embodiment, the B block comprises 25-65 wt % VDF and 15-60 wt % HFP; or even 35-60 wt % VDF and 25-50 wt % HFP. Monomers, in addition, to those mentioned above, may be included in the A and/or B blocks. Generally, the weight average of the A block and B block are independently selected from at least 1000, 5000, 10000, or even 25000 daltons; and at most 400000, 600000, or even 800000 daltons. Such block copolymers are disclosed in WO 2017/013379 (Mitchell et al.); and U.S. Provisional Appl. Nos. 62/447675, 62/447636, and 62/447664, each filed 18 Jan 2017.

The fluorinated elastomeric gum of the present disclosure comprises cure sites, which act as reaction sites for crosslinking the fluoropolymer to form a fluoroelastomer. Typically, the fluorinated elastomeric gum comprises at least 0.05, 0.1, 0.5, 1, or even 2% by mole of cure sites and at most 5, or even 10 % by mole of cure sites versus moles of fluorinated elastomeric gum.

In the present disclosure, the fluorinated elastomeric gum may be polymerized in the presence of a chain transfer agent and/or cure site monomers to introduce the cure sites into the fluorinated elastomeric gum.

Exemplary chain transfer agents include: an iodo-chain transfer agent, and a bromo-chain transfer agent. For example, suitable iodo-chain transfer agent in the polymerization include the formula of RIₓ, where (i) R is a perfluoroalkyl or chloroperfluoroalkyl group having 3 to 12 carbon atoms; and (ii) x = 1 or 2. The iodo-chain transfer agent may be a perfluorinated iodo-compound. Exemplary iodo-perfluoro-compounds include 1,3-diiodoperfluoropropane, 1,4-diiodoperfluorobutane, 1, 6-diiodoperfluorohexane, 1,8-diiodoperfluorooctane, 1,10-diiodoperfluorodecane, 1,12-diiodoperfluorododecane, 2-iodo-1,2-dichloro-l,1,2-trifluoroethane, 4-iodo-1,2,4-trichloroperfluorobutan, and mixtures thereof. In some embodiments, the bromine is derived from a brominated chain transfer agent of the formula: RBrₓ, where (i) R is a perfluoroalkyl or chloroperfluoroalkyl group having 3 to 12 carbon atoms; and (ii) x = 1 or 2. The chain transfer agent may be a perfluorinated bromo-compound.

Cure-site monomers, if used, comprise at least one of a bromine, iodine, and/or nitrile cure moiety.

In one embodiment, the cure site monomers may be derived from one or more compounds of the formula: (a) CX₂=CX(Z), wherein: (i) X each is independently H or F; and (ii) Z is I, Br, R*_{f}*-U wherein U=I or Br and R*_{f}*=a perfluorinated or partially perfluorinated alkylene group optionally containing O atoms; or (b) Y(CF₂)_{q}Y, wherein: (i) Y is Br or I or Cl and (ii) q=1-6. In addition, non-fluorinated bromo-or iodo-olefins, e.g., vinyl iodide and allyl iodide, can be used. In some embodiments, the cure site monomers are derived from compounds such as CH₂=CHI, CF₂=CHI, CF₂=CFI, CH₂=CHCH₂I, CF₂=CFCF₂I, ICF₂CF₂CF₂CF₂I, CH₂=CHCF₂CF₂I, CF₂=CFCH₂CH₂I, CF₂=CFCF₂CF₂I, CH₂=CH(CF₂)₆CH₂CH₂I, CF₂=CFOCF₂CF₂I, CF₂=CFOCF₂CF₂CF₂I, CF₂=CFOCF₂CF₂CH₂I, CF₂=CFCF₂OCH₂CH₂I, CF₂=CFO(CF₂)₃-OCF₂CF₂I, CH₂=CHBr, CF₂=CHBr, CF₂=CFBr, CH₂=CHCH₂Br, CF₂=CFCF₂Br, CH₂=CHCF₂CF₂Br, CF₂=CFOCF₂CF₂Br, CF₂=CFCl, CF₂=CFCF₂Cl, or combinations thereof.

In another embodiment, the cure site monomers comprise nitrile-containing cure moieties. Useful nitrile-containing cure site monomers include nitrile-containing fluorinated olefins and nitrile-containing fluorinated vinyl ethers, such as: perfluoro(8-cyano-5-methyl-3,6-dioxa-1-octene); CF₂=CFO(CF₂)_{L}CN wherein L is an integer from 2 to 12; CF₂=CFO(CF₂)ᵤOCF(CF₃)CN wherein u is an integer from 2 to 6; CF₂=CFO[CF₂CF(CF₃)O]_{q}(CF₂O)_{y}CF(CF₃)CN; CF₂=CFO[CF₂CF(CF₃)O]_{q}(CF₂)_{y}OCF(CF₃)CN wherein q is an integer from 0 to 4 and y is an integer from 0 to 6; CF₂=CF[OCF₂CF(CF₃)]ᵣO(CF₂)ₜCN wherein r is 1 or 2, and t is an integer from 1 to 4; and derivatives and combinations of the foregoing. Examples of a nitrile-containing cure site monomer include CF₂=CFO(CF₂)₅CN, CF₂=CFOCF₂CF(CF₃)OCF₂CF₂CN, CF₂=CFOCF₂CF(CF₃)OCF₂CF(CF₃)CN, CF₂=CFOCF₂CF₂CF₂OCF(CF₃)CN, CF₂=CFOCF₂CF(CF₃)OCF₂CF₂CN; and combinations thereof.

### Peroxide

The co-agents of Formula I disclosed herein or salts thereof are used in conjunction with a peroxide to cure the fluorinated elastomeric gum. Peroxide curatives include organic or inorganic peroxides. Organic peroxides are preferred, particularly those that do not decompose during dynamic mixing temperatures.

In many cases it is preferred to use a tertiary butyl peroxide having a tertiary carbon atom attached to a peroxy oxygen.

Examples of the organic peroxide include benzoyl peroxide, dicumyl peroxide, di-tert-butyl peroxide, 2,5-di-methyl-2,5-di-tert-butylperoxyhexane, 2,4-dichlorobenzoyl peroxide, 1,1-bis(tert-butylperoxy)-3,3,5-trimethylchlorohexane, tert-butyl peroxy isopropylcarbonate (TBIC), tert-butyl peroxy 2-ethylhexyl carbonate (TBEC), tert-amyl peroxy 2-ethylhexyl carbonate, tert-hexylperoxy isopropyl carbonate, carbonoperoxoic acid, O,O'-1,3-propanediyl OO,OO'-bis(1,1-dimethylethyl) ester, tert-butylperoxy benzoate, t-hexyl peroxy-2-ethylhexanoate, t-butyl peroxy-2-ethylhexanoate, di(4-methylbenzoyl) peroxide, laurel peroxide, cyclohexanone peroxide, and combinations thereof. Other suitable peroxide curatives are listed in U.S. Pat. No. 5,225,504 (Tatsu et al.).

The amount of peroxide used generally will be at least 0.1, 0.2, 0.4, 0.6, 0.8, 1, 12, or even 1.5; and at most 2, 2.25, 2.5, 2.75, 3, 3.5, 4, 4.5, 5, or even 5.5 parts by weight per 100 parts of the fluorinated elastomeric gum.

In one embodiment, the compositions of the present disclosure (and the cured articles derived therefrom) are substantially free of a traditional co-agent, meaning the composition comprises less than 0.1, or even 0.05 parts by weight per 100 parts by weight per fluorinated elastomeric gum or even below detection of a traditional co-agent. As used herein, a traditional co-agent includes (a) non-fluorinated, polyunsaturated compounds having 2 or more terminal unsaturation sites or (b) partially fluorinated compounds comprising two terminal unsaturation sites. Exemplary non-fluorinated, polyunsaturated compounds having terminal unsaturation sites include: tri(methyl)allyl isocyanurate (TMAIC), triallyl isocyanurate (TAIC), tri(methyl)allyl cyanurate, poly-triallyl isocyanurate (poly-TAIC), triallyl cyanurate (TAC), xylylene-bis(diallyl isocyanurate) (XBD), N,N'-m-phenylene bismaleimide, diallyl phthalate, tris(diallyl amine)-s-triazine, triallyl phosphite, 1,2-polybutadiene, ethyleneglycol diacrylate, diethyleneglycol diacrylate, and combinations thereof. Exemplary partially fluorinated compounds comprising two terminal unsaturation sites include: CH₂=CH-R_{f1}-CH=CH₂ wherein R_{f1} may be a perfluoroalkylene of 1 to 8 carbon atoms.

In one embodiment, in addition to the co-agent of Formula I disclosed herein, a traditional co-agent may be employed as well. In one embodiment, the composition of the present disclosure comprises 0.5 part by weight or more, 1 part by weight or more, 5 weight by mass or more, 10 parts by weight or more, or 15 parts by weight or more of a traditional co-agent; and 60 parts by weight or less, 40 parts by weight or less, 30 parts by weight or less, or 20 parts by weight or less of the traditional co-agent, per 100 parts by weight of the fluorinated elastomeric gum. In one embodiment, the addition of the co-agent of Formula I with a traditional co-agent may have better performance (such as thermal resistivity) than if the traditional co-agent was used alone, assuming the same molar amount of allyl groups. In one embodiment, when both a co-agent of Formula I and a traditional co-agent are used, the amount of co-agent of Formula I is the same or greater than the amount of traditional co-agent.

In one embodiment, the composition of the present disclosure is substantially free (wherein the composition comprises less than 0.1 parts by weight) of a co-agent comprising cyclic and/or aromatic groups.

The composition of the present disclosure (and the cured articles derived therefrom) can also contain a wide variety of additives of the type normally used in the preparation of elastomeric compositions, such as pigments, fillers (such as carbon black), pore-forming agents, and those known in the art.

Additives may include: an organic or inorganic fillers such as clay, silica (SiO₂), alumina, iron red, talc, diatomaceous earth, barium sulfate, wollastonite (CaSiO₃), calcium carbonate (CaCO₃), calcium fluoride, titanium oxide, iron oxide and carbon black fillers, a polytetrafluoroethylene powder, PFA (TFE/perfluorovinyl ether copolymer) powder, an electrically conductive filler, a heat-dissipating filler may be added as an optional component to the composition. Those skilled in the art are capable of selecting specific fillers at required amounts to achieve desired physical characteristics in the cured elastomer. The filler components may result in a fluoroelastomer that is capable of retaining a preferred elasticity and physical tensile, as indicated by an elongation and tensile strength value, while retaining desired properties such as retraction at lower temperature (TR-10).

In one embodiment, the composition comprises equal to or less than 50, 40, 30, 20, 15, or even 10% by weight of the filler per weight of the fluorinated elastomeric gum.

Conventional adjuvants may also be incorporated into the composition of the present disclosure to enhance the properties of the resulting composition. For example, acid acceptors may be employed to facilitate the cure and thermal stability of the composition. Suitable acid acceptors may include magnesium oxide, lead oxide, calcium oxide, calcium hydroxide, dibasic lead phosphite, zinc oxide, barium carbonate, strontium hydroxide, calcium carbonate, hydrotalcite, alkali stearates, magnesium oxalate, or combinations thereof. The acid acceptors are preferably used in amounts ranging from 1 to 20 parts by weight per 100 parts by weight of the polymer.

The curable compositions may be prepared by mixing the fluorinated elastomeric gum, and the co-agent of Formula I, along with the other components (e.g., the peroxide, a traditional co-agent, and/or the additional additives) in conventional rubber processing equipment to provide a solid mixture, i.e. a solid polymer containing the additional ingredients, also referred to in the art as a "compound". This process of mixing the ingredients to produce such a solid polymer composition containing other ingredients is typically called "compounding". Such equipment includes rubber mills, internal mixers, such as Banbury mixers, and mixing extruders. The temperature of the mixture during mixing typically will not rise above 120°C. During mixing the components and additives are distributed uniformly throughout the resulting fluorinated polymer "compound" or polymer sheets. The "compound" can then be extruded or pressed in a mold, e.g., a cavity or a transfer mold and subsequently be oven-cured. In an alternative embodiment curing can be done in an autoclave.

The mixture may then be processed and shaped such as by extrusion or molding to form an article of various shapes such as sheet, a hose, a hose lining, an o-ring, a gasket, or a seal composed of the composition of the present disclosure. The shaped article may then be heated to cure the gum composition and form a cured elastomer article.

Pressing of the compounded mixture (i.e., press cure) is typically conducted at a temperature of 120-220°C, preferably 140-200°C, for a period of 1 minute to 15 hours, usually for 1-15 minutes. A pressure of 700-20,000 kPa, preferably 3400- 6800 kPa, is typically used in molding the composition. The molds first may be coated with a release agent and prebaked.

The molded vulcanizate can be post cured in an oven at a temperature of between 140°C and 250°C, between 180°C and 250°C, or between 200°C and 235°C for a period of 1-24 hours or more, depending on the cross-sectional thickness of the sample. For thick sections, the temperature during the post cure is usually raised gradually from the lower limit of the range to the desired maximum temperature. The maximum temperature used is preferably 260°C, and is held at this value for 1 hour or more. Curing is preferably carried out under pressure. For example, pressures from 10 to 100 bar may be applied. A post curing cycle may be applied to ensure the curing process is fully completed. Post curing may be carried out at a temperature between 170°C and 250°C for a period of 1 to 24 hours.

In one embodiment, the cured fluoroelastomers have an elongation at break of greater than 145, 150, 160, or even at 180% when the cured samples are tested by tensile strength and elongation at break are tested in accordance with ASTM 412-06a.

In one embodiment, the cured fluoroelastomers have a tear resistance which is improved over the same cured fluoroelastomer made with a traditional co-agent having the same molar amount of allyl groups when tested by the Tear C test disclosed below.

Compositions of the present disclosure may be used in articles, such as a molded part, a hose, a gasket, or a seal.

### EXAMPLES

Unless otherwise noted, all parts, percentages, ratios, etc. in the examples and the rest of the specification are by weight, and all reagents used in the examples were obtained, or are available, from general chemical suppliers such as, for example, Sigma-Aldrich Company, Saint Louis, Missouri, or may be synthesized by conventional methods.

The following abbreviations are used in this section: mL=milliliters, g=grams, lb=pounds, min=minutes, h=hours, NMR=nuclear magnetic resonance, eq=equivalent, mmHg=millimeters mercury, °C=degrees Celsius, °F=degrees Farenheit, phr=parts per hundred rubber, MPa = mega Pascal, psi = pounds per square inch, N·m = Newton meter, and kN/m = kilo Newton per meter. Abbreviations for materials used in this section, as well as descriptions of the materials, are provided in Table 1.

**Table 1**

| Material | Details |
|---|---|
| Fluoropolymer | Polymer 3 of WO 2017/011379 (Mitchell et al.) |
| carbon black | Carbon black commercially available from Cancarb Ltd, Medicine Hat, AB, Canada under the trade designation "THERMAX N990 MT". |
| TAIC | Triallyl-isocyanurate commercially available under the trade designation "TAIC" from Nippon Kasei Chemical Co. Ltd., Tokyo, Japan |
| Peroxide | 2,5-dimethyl-2,5-di(t-butylperoxy)-hexane, about 50% active on an inert carrier, available under the trade designation "VAROX DBPH-50" from Vanderbilt Chemicals, LLC., Norwalk, CT. |
| Co-agent 1 | A co-agent, octafluorodicarboxamide tetraallyl, (CH₂=CHCH₂)₂NCOC₄F₈CON(CH₂CH=CH₂)₂, prepared as described in PE-1 |
| Co-agent 2 | A co-agent, octafluorobutanedisulfonamide tetraallyl, (CH₂=CHCH₂)₂NSO₂C₄F₈SO₂N(CH₂CH=CH₂)₂, prepared as described in PE-2 |
| Co-agent 3 | A co-agent, octafluoro-dioxa-octanedicarboxamide tetraallyl, (CH₂=CHCH₂)₂NCOCF₂OC₂F₄OCF₂CON(CH₂CH=CH₂)₂, prepared as described in PE-3 |
| Co-agent 4 | A co-agent, nonafluorobutanesulfonamide diallyl, C₄F₉SO₂N(CH₂CH=CH₂)₂, prepared as described in PE-4 |
| Co-agent 5 | A co-agent, hexafluoropropanecarboxamide/sulfonamide tetraallyl, (CH₂=CHCH₂)₂NCOC₃F₆SO₂N(CH₂CH=CH₂)₂, prepared as described in PE-5 |
| Co-agent 6 | A co-agent, hexafluoropropanedisulfonamide diallyl, CH₂=CHCH₂NHSO₂C₃F₆SO₂NHCH₂CH=CH₂, prepared as described in PE-6 |
| Co-agent 7 | A co-agent, oxyoctafluorodiethanedisulfonamide tetraallyl, (CH₂=CHCH₂)₂NSO₂C₂F₄OC₂F₄SO₂N(CH₂CH=CH₂)₂, prepared as described in PE-7 |

### Preparative Example 1 (PE-1): Preparation of co-agent octafluorobutanedicarboxamide tetraallyl, (CH₂=CHCH₂)₂NCOC₄F₈CON(CH₂CH=CH₂)₂, FC6DATA

To a 500 mL 3-neck round bottom flask equipped with a mechanical stirrer, was added 50 g FCOC₄F₈COF (perfluorohexanedioyl difluoride, 0.17 mol, available from Exfluor Reseach, Austin, TX), 100 g methyl-tert-butyl ether, and 35 g triethylamine (0.35 mol). The mixture was stirred at room temperature (i.e., about 25 °C). 34 g of diallyl amine (0.35 mol) was added over the course of 30 min, which caused the temperature to increase to 50 °C. The mixture then was stirred for 1 h, then allowed to cool to room temperature. Then 200 g of distilled water was added. The top organic phase was atmospherically distilled to remove volatiles and the product was vacuum distilled providing 49 g of (CH₂=CHCH₂)₂NCOC₄F₈CON(CH₂CH=CH₂)₂, which had a boiling point of 158 °C at 2 mmHg (0.27 kPa), resulting in a 65% yield. NMR was used to confirm the compound.

### Preparative Example 2 (PE-2): Preparation of co-agent octafluorobutanedisulfonamide tetraallyl, (CH₂=CHCH₂)₂NSO₂C₄F₈SO₂N(CH₂CH=CH₂)₂, FC4DSATA

To a 250 mL 3-neck round bottom flask with a mechanical stirrer, was added 160 g diallyl amine (1.65 mol). The liquid was stirred at room temperature. 100 g FSO₂C₄F₈SO₂F (0.27 mol, which was made by electrochemical fluorination of 1,4-butane disulfonyl chloride as described is US 2,732,398) was added over the course of 30 min, which caused the temperature to increase to 40 °C. The mixture then was stirred and heated to 95 °C for 20 h. The reaction mixture was cooled to room temperature, followed by the addition of 150 g distilled water. The top organic phase was atmospherically distilled to remove volatiles and followed by vacuum distillation of the product to provide 85 g (CH₂=CHCH₂)₂NSO₂C₄F₈SO₂N(CH₂CH=CH₂)₂, which had a boiling point of 182 °C at 3mmHg (0.39 kPa) resulting in a 60% yield. NMR was used to confirm the compound.

### Preparative Example 3 (PE-3): Preparation of co-agent octafluoro-dioxa-octanedicarboxamide tetraallyl, (CH₂=CHCH₂)₂NCOCF₂OC₂F₄OCF₂CON(CH₂CH=CH₂)₂, FTEGDATA

To a 1 L 3-neck round bottom flask equipped with a mechanical stirrer, was added 69 g triethylamine (0.68 mol), 66 g diallyl amine (0.68 mol) and 210 g methyl-tert-butyl ether. The mixture was stirred at room temperature. 100 g FCOCF₂OC₂F₄OCF₂COF (perfluoro-3,6-dioxaoctanedioyl difluoride, 0.31 mol, available from Exfluor Research, Austin, TX) was added over the course of 30 min, which caused the temperature to increase to about 44 °C. The mixture was stirred for 1 h, then 200 g of distilled water was added. The organic phase was atmospherically distilled to remove volatiles and the product was vacuum distilled to obtain 130 g (CH₂=CHCH₂)₂NCOCF₂OC₂F₄OCF₂CON(CH₂CH=CH₂)₂, which had a boiling point at 145 °C at 4 mmHg (0.53 kPa) resulting in an 88% yield. NMR was used to confirm the compound.

### Preparative Example 4 (PE-4): Preparation of co-agent nonafluorobutanesulfonamide diallyl, C₄F₉SO₂N(CH₂CH=CH₂)₂, C4MSDA

To a 250 mL 3-neck round bottom flask with a mechanical stirrer, was added 50 g diallyl amine (0.5 mol). The liquid was stirred at room temperature. 50 g C₄F₉SO₂F (0.17 mol, made by electrochemical fluorination as described is US 2,732,398) was added over the course of 30 min, which cause the reaction temperature to increase to 27 °C. The mixture was heated to 95 °C and allowed to react for 20 h. The reaction mixture was then cooled to room temperature, and a solution of 30 g NaCl in 100 g distilled water and 90 g methyl-tert-butyl ether was added and then mixed for 10 min. The top organic phase was removed and washed with 100 g distilled water. Volatiles were removed from the organic phase by rotary evaporation and vacuum distillation produced 37 g C₄F₉SO₂N(CH₂CH=CH₂)₂, which had a boiling point of 99 °C at 11 mmHg (1.47 kPa), resulting in a 60% yield. NMR was used to confirm the compound.

### Preparative Example 5 (PE-5): Preparation of hexafluoropropanecarboxamide/sulfonamide tetraallyl, (CH₂=CHCH₂)₂NCOC₃F₆SO₂N(CH₂CH=CH₂)₂, C3ASTA

To a 500 mL 3-neck round bottom flask with a mechanical stirrer, was added 207 g diallyl amine (2.14 mol). The liquid was stirred at room temperature. 100 g FCO₃F₆SO2F (0.36 mol, made by electrochemical fluorination as described is US 6,624,328) was added over the course of 1 h, which cause the temperature to increase to 51 °C. The mixture was heated to 95 °C and allowed to react for 20 h. The reaction mixture then was cooled to room temperature and 150 g of distilled water was added. The top phase was vacuum distilled to remove volatiles obtaining 79 g (CH₂=CHCH₂)₂NCOC₃F₆CSO₂N(CH₂CH=CH₂)₂ which had a boiling point of 192 °C at 10 mmHg (1.33 kPa), resulting in a 51% yield. NMR was used to confirm the compound.

### Preparative Example 6 (PE-6): Preparation of co-agent hexafluoropropanedisulfonamide diallyl, CH₂=CHCH₂NHSO₂C₃F₆SO₂NHCH₂CH=CH₂, C3DSDA

To a 1 L 3-neck round bottom flask with a mechanical stirrer, was added 108 g allylamine (1.9 mol) and 175 g methyl-tert-butyl ether. The mixture was stirred at room temperature. 100 g FSO₂C₃F₆SO₂F (0.32 mol, made by electrochemical fluorination of 1,3-propane disulfonyl chloride as described is US 2,732,398) was added over the course of 1 h, which caused the temperature to increase to 53 °C. The mixture was kept at 40 °C and allowed to react for 1 h. The reaction mixture was cooled to room temperature, then 250 g of distilled water was added. The organic phase was isolated by rotary evaporation, yielding 118 g of semi-solid product. Recrystallization was done by adding 100 g of toluene to the semi-solid product, heating to 90 °C, and then cooling the solution to 25 °C. The solution was then filtrated, which yielded 52 g of a white solid, CH₂=CHCH₂NHSO₂C₃F₆SO₂NHCH₂CH=CH₂, which had a melting point of 164 °C, resulting in a 42% yield. NMR was used to confirm the compound.

### Preparative Example 7 (PE-7): Preparation of co-agent oxyoctafluorodiethanedisulfonamide tetraallyl, (CH₂=CHCH₂)₂NSO₂C₂F₄OC₂F₄SO₂N(CH₂CH=CH₂)₂, EC4DSATA

To a 250 mL 3-neck round bottom flask with a mechanical stirrer, was added 20 g diallyl amine (0.21 mol). The liquid was stirred at room temperature. 10 g FSO₂C₂F₄OC₂F₄SO₂F (0.03 mol, available from SynQuest Laboratories Inc, Alachua, FL, USA) was charged into the flask all at once. The mixture was heated to 95 °C and allowed to react for 20 h. The reaction mixture was cooled to room temperature and vacuum was used to remove excess reagent. The product was heated while vacuum distilling, yielding 5.2 g (CH₂=CHCH₂)₂NSO₂C₂F₄OC₂F₄SO₂N(CH₂CH=CH₂)₂, which had a boiling point of 178 °C at 3 mmHg (0.40kPa), resulting in a 37% yield. NMR was used to confirm the compound.

### Characterization Methods

### Melting point measurement and Glass transition

Melting point (T*ₘ*) and glass transition temperature (T*_{g}*) were determined in accordance with ASTM D 793-01 and ASTM E 1356-98 with a differential scanning calorimetry (DSC) (DSC Q2000 available from TA Instruments, New Castle, DE) under a nitrogen flow. A DSC scan was obtained from -80 °C to 325 °C at 10 °C / min scan rate.

### Cure Rheology

Cure rheology tests were carried out using uncured, compounded samples using a rheometer marketed under the trade designation PPA 2000 by Alpha technologies, Akron, OH, in accordance with ASTM D 5289-93a at 177 °C, no pre-heat, 12 min elapsed time, and a 0.5 degree arc. Both the minimum torque (M_{L}) and highest torque attained during a specified period of time when no plateau or maximum torque (M_{H}) was obtained were measured. Also measured were the time for the torque to increase 2 units above M_{L} (t_{S}2), the time for the torque to reach a value equal to M_{L} + 0.1(M_{H} - M_{L}), (t'10), the time for the torque to reach a value equal to M_{L} + 0.5(M_{H} - M_{L}), (t'50), and the time for the torque to reach M_{L} + 0.9(M_{H} - M_{L}), (t'90).

### Tensile and Tear C

Tensile data was gathered from both press and post cured samples cut to Die D specifications at room temperature in accordance with ASTM 412-06A. Tensile data at temperature was measured on Die D dumbells. Tear C data was gathered on post cured sheets in accordance with ASTM D624-00(2012).

### Molding O-rings and Compression Set

O-rings (214, AMS AS568) were molded at 177 °C for 10 min. The press cured O-rings were post-cured at 232 °C for 4 h. The press cured and post cured O-rings were tested for compression set for 70 h at 200 °C in accordance with ASTM D 395-03 Method B and ASTM D 1414-94 with 25 % initial deflection. Results are reported as percentages.

### Compounding

200 g polymer batches were compounded with the amounts of materials as listed in Table 2 on a two-roll mill.

**Table 2**

| | CE-1 | EX-1 | EX-2 | EX-3 | EX-4 | CE-2 | EX-5 | CE-3 | EX-7 |
|---|---|---|---|---|---|---|---|---|---|
| Formulation (phr) | | | | | | | | | |
| Fluoroelastomer gum | 100 | 100 | 100 | 100 | 100 | 100 | 100 | 100 | 100 |
| N990 | 30 | 30 | 30 | 30 | 30 | 30 | 30 | 30 | 30 |
| TAIC | 3 | | | | 1.5 | | | | |
| DBPH-50 | 2 | 2 | 2 | 2 | 2 | 2 | 2 | 2 | 2 |
| Co-agent 1 | | 3.6 | | | | | | | |
| Co-agent 2 | | | 4.65 | | 1.5 | | | | |
| Co-agent 3 | | | | 4.33 | | | | | |
| Co-agent 4 | | | | | | 6.8 | | | |
| Co-agent 5 | | | | | | | 3.9 | | |
| Co-agent 6 | | | | | | | | 7.0 | |
| Co-agent 7 | | | | | | | | | 4.8 |

| Cure rheology | | | | | | | | | |
|---|---|---|---|---|---|---|---|---|---|
| ML, Minimum Torque, inch-lb (N·m) | 4.6 (0.5) | 4.2 (0.5) | 3.8 (0.4) | 4.6 (0.5) | 4.3 (0.5) | 3.6 (0.4) | 4.2 (0.5) | 4.0 (0.5) | 3.8 (0.5) |
| MH, Maximum Torque, inch-lb (N·m) | 43.3 (4.9) | 20.7 (4.9) | 25.0 (2.8) | 15.1 (1.7) | 33.4 (3.8) | 5.7 (0.6) | 21.5 (2.4) | 4.1 (0.5) | 25 (2.8) |
| Delta torque inch-lb (N·m) | 38.7 (4.4) | 16.5 (1.9) | 21.2 (2.4) | 10.5 (1.2) | 29.2 (3.3) | 2.1 (0.2) | 17.3 (2.0) | 0.1 (0) | 21.1 (2.3) |
| t'50, Time to 50% cure - min | 0.7 | 1.0 | 1.2 | 1.2 | 1.3 | 0.1 | 1.4 | 11.9 | 1.3 |
| t'90, Time to 90% cure - min | 1.1 | 2.7 | 3.2 | 3.8 | 3.5 | 6.2 | 3.9 | 11.9 | 4.3 |
| tan δ ML | 0.66 | 0.61 | 1.14 | 0.41 | 0.43 | 1.35 | 0.39 | 0.49 | 0.87 |
| tan δ MH | 0.071 | 0.156 | 0.112 | 0.191 | 0.098 | 0.383 | 0.13 | 0.477 | 0.12 |

| Typical Physical Properties | | | | | | | | | |
|---|---|---|---|---|---|---|---|---|---|
| Press Cure @ 177 °C (350°F), 10 min | | | | | | | | | |
| Tensile, psi (MPa) | 4588 (31.7) | 3439 (23.7) | NM | 2221 (15.3) | 3387 (23.4) | Not enough cure | 3119 (21.5) | Not enough cure | 3371 (23.3) |
| Elongation at break, % | 163 | 210 | NM | 356 | 219 | NM | 136 | NM | 132 |
| 100% Modulus, psi (MPa) | 2990 (20.6) | 2049 (14.1) | NM | 1301 (9.0) | 1712 (11.8) | NM | 2394 (16.5) | NM | 2661 (18.4) |
| Hardness, Shore A | 92 | 92 | NM | 92 | 94 | NM | 94 | NM | 94 |

| Post Cure @ 232 °C (450°F), 4 h | | | | | | | | | |
|---|---|---|---|---|---|---|---|---|---|
| Tensile, psi (MPa) | 4605 (31.8) | 4280 (29.5) | 4479 (30.9) | 3636 (25.1) | 4921 (34.0) | NM | 4614 (31.8) | NM | 4044 (27.9) |
| Elongation at break, % | 117 | 198 | 200 | 243 | 164 | NM | 194 | NM | 153 |
| Stress at 100% strain, psi (MPa) | 3766 (26.0) | 2241 (15.5) | 2388 (16.5) | 1677 (11.6) | 2954 (20.4) | NM | 2586 (17.8) | NM | 2639 (27.9) |
| Hardness, Shore A | 93 | 93 | 92 | 93 | 94 | NM | 92 | NM | 92 |

| Compression Set at 70 h @200 °C | | | | | | | | | |
|---|---|---|---|---|---|---|---|---|---|
| press cure | 55 | 108 | 100 | 85 | 109 | NM | >100 | NM | >100 |
| post cure | 49 | 89 | 83 | 73 | 97 | NM | 92 | NM | 89 |
| | | | | | | | | | |
| 23 Die C Tear Strength (Post Cured), kN/m | 62 | 66 | 64 | 66 | 61 | NM | NM | NM | NM |
| 150C Die C Tear Strength (Post Cured) kN/m | 12 | 11 | 11 | NM | 11 | NM | NM | NM | NM |

| | | | | | | | | | |
|---|---|---|---|---|---|---|---|---|---|
| NM= not measured | | | | | | | | | |

## Claims

1. A composition comprising:
a. a fluorinated elastomeric gum comprising at least 0.05% by weight of a cure site, wherein the cure site comprises at least one of bromine, iodine, nitrile, or combinations thereof;
b. a co-agent of Formula I or a salt thereof, wherein Formula I is
(CH₂=CHCH₂)₂-L¹-Rf-L²-(CH₂CH=CH₂)ₙ(H)₂₋ₙ
wherein L¹ is -NSO₂- or -NCO-; L² is -SO₂N- or -CON-; n is 1 or 2; and Rf is a divalent perfluorinated carbon group, optionally comprising at least one ether linkage.

2. The composition of claim 1, wherein the salt of Formula I comprises a salt forming cation with a valence of 1, 2, or 3.

3. The composition of any one of the previous claims, wherein the co-agent comprises at least one of:
(CH₂=CHCH₂)₂NSO₂C₃F₆SO₂N(CH₂CH=CH₂)₂;
(CH₂=CHCH₂)₂NSO₂C₄F₈SO₂N(CH₂CH=CH₂)₂;
(CH₂=CHCH₂)₂NSO₂C₆F₁₀SO₂N(CH₂CH=CH₂)₂;
(CH₂=CHCH₂)₂NSO₂C₂F₄OC₂F₄SO₂N(CH₂CH=CH₂)₂;
(CH₂=CHCH₂)₂NCOC₄F₈CON(CH₂CH=CH₂)₂;
(CH₂=CHCH₂)₂NCOCF₂OC₂F₄OCF₂CON(CH₂CH=CH₂)₂;
(CH₂=CHCH₂)₂NCOCF₂OC₂F₄OC₂F₄OCF₂CON(CH₂CH=CH₂)₂;
(CH₂=CHCH₂)₂NCOC₃F₆SO₂N(CH₂CH=CH₂)₂;
CH₂=CHCH₂NHSO₂C₃F₆CON(CH₂CH=CH₂)₂
and salts thereof; and
(CH₂=CHCH₂)₂NSO₂C₃F₆CONHCH₂CH=CH₂
and salts thereof

4. The composition of any one of the previous claims, wherein the salt is selected from at least one of: NH₄⁺, H₃O⁺, Na⁺, Li⁺, Cs⁺, Ca⁺², K⁺, Mg⁺², Zn⁺², and Cu⁺², N(CH₃)₄⁺, NH₂(CH₃)₂⁺, N(CH₂CH₃)₄⁺, NH(CH₂CH₃)₃⁺, NH(CH₃)₃⁺, ((CH₃CH₂CH₂CH₂)₄)P⁺, or combinations thereof.

5. The composition of any one of claims 1, 2 or 4, wherein Rf is branched or cyclic.

6. The composition of any one of the previous claims, wherein the fluorinated elastomeric gum comprises at least one of: (i) copolymers comprising TFE and a perfluoroalkyl vinyl ether monomeric units; (ii) copolymers comprising TFE and a perfluoroalkoxy vinyl ether monomeric units; (iii) copolymers comprising TFE and propylene monomeric units; (iv) copolymers comprising TFE, propylene, and VDF monomeric units; (v) copolymers comprising VDF and HFP monomeric units; (vi) copolymers comprising TFE, VDF, and HFP monomeric units; (vii) copolymers comprising VDF and perfluoroalkyl vinyl ether monomeric units; (viii) copolymers comprising CTFE and VDF monomeric units; (ix) copolymers comprising TFE and VDF monomeric units; (x) copolymers comprising TFE, VDF and perfluoroalkyl vinyl ether monomeric units; and (xi) combinations thereof.

7. The composition of any one of claims 1-5, wherein the fluorinated elastomeric gum is a block copolymer comprising at least one A block and at least one B block.

8. The composition of any one of the previous claims, wherein the fluorinated elastomeric gum comprises at most 5 % by weight of the cure-site.

9. The composition of any one of the previous claims, comprising at least 3 to at most 10 parts by weight of the co-agent per 100 parts by weight of the fluorinated elastomeric gum.

10. The composition of any one of the previous claims, further comprising a peroxide.

11. The composition of claim 10, comprising at least 0.25 parts by weight to no more than 10 parts by weight of the peroxide per 100 parts by weight of the fluorinated elastomeric gum.

12. The composition according to any one of the previous claims further comprising a filler, optionally, wherein the composition comprises less than 50% by weight of the filler based on the weight of the fluorinated elastomeric gum.

13. An article comprising the cured composition according to any one of claims 1-12.

14. A method of curing a fluorinated elastomeric gum, the method comprising:
obtaining a fluorinated elastomeric gum comprising cure sites, wherein the cure site comprises at least one of Br, I or nitrile;
curing the fluorinated elastomeric gum with a peroxide and a co-agent, wherein the co-agent is according to Formula I:
(CH₂=CHCH₂)₂-L¹-Rf-L²-(CH₂CH=CH₂)ₙ(H)₂₋ₙ
wherein L¹ is -NSO₂- or -NCO-; L² is -SO₂N- or -CON-; n is 1 or 2; and Rf is a divalent perfluorinated carbon group, optionally comprising at least one ether linkage.

15. The composition according to any one of claims 1-12, wherein the composition comprises less than 0.1 parts by weight of a non-fluorinated, polyunsaturated compound having two or more terminal unsaturation sites per 100 parts by weight per fluorinated elastomeric gum.

## Patentansprüche

1. Eine Zusammensetzung, umfassend:
a. einen fluorierten Elastomerkautschuk, umfassend zu mindestens 0,05 Gew.-% eine Härtungsstelle, wobei die Härtungsstelle mindestens eines von Brom, lod, Nitril oder Kombinationen davon umfasst;
b. ein Co-Mittel von Formel I oder ein Salz davon, wobei Formel I
(CH₂=CHCH₂)₂-L¹-Rf-L²-(CH₂CH=CH₂)ₙ(H)₂₋ₙ ist,
wobei L¹ -NSO₂₋ oder -NCO- ist; L² -SO₂N- oder -CON- ist; n 1 oder 2 ist; und Rf eine zweiwertige perfluorierte Kohlenstoffgruppe ist, gegebenenfalls umfassend mindestens eine Etherbindung.

2. Die Zusammensetzung nach Anspruch 1, wobei das Salz der Formel I ein salzbildendes Kation mit einer Valenz von 1, 2 oder 3 umfasst.

3. Die Zusammensetzung nach einem der vorstehenden Ansprüche, wobei das Co-Mittel mindestens eines umfasst von:
(CH₂=CHCH₂)₂NSO₂C₃F₆SO₂N(CH₂CH=CH₂)₂;
(CH₂=CHCH₂)₂NSO₂C₄F₈SO₂N(CH₂CH=CH₂)₂;
(CH₂=CHCH₂)₂NSO₂C₆F₁₀SO₂N(CH₂CH=CH₂)₂;
(CH₂=CHCH₂)₂NSO₂C₂F₄OC₂F₄SO₂N(CH₂CH=CH₂)₂;
(CH₂=CHCH₂)₂NCOC₄F₈CON(CH₂CH=CH₂)₂;
(CH₂=CHCH₂)₂NCOCF₂OC₂F₄OCF₂CON(CH₂CH=CH₂)₂;
(CH₂=CHCH₂)₂NCOCF₂OC₂F₄OC₂F₄OCF₂CON(CH₂CH=CH₂)₂;
(CH₂=CHCH₂)₂NCOC₃F₆SO₂N(CH₂CH=CH₂)₂;
CH₂=CHCH₂NHSO₂C₃F₆CON(CH₂CH=CH₂)₂
und Salzen davon; und
(CH₂=CHCH₂)₂NSO₂C₃F₆CONHCH₂CH=CH₂
und Salzen davon

4. Die Zusammensetzung nach einem der vorstehenden Ansprüche, wobei das Salz ausgewählt ist aus mindestens einem von: NH₄⁺, H₃O⁺, Na⁺, Li⁺, Cs⁺, Ca⁺², K⁺, Mg⁺², Zn⁺², and Cu⁺², N(CH₃)₄⁺, NH₂(CH₃)₂⁺, N(CH₂CH₃)₄⁺, NH(CH₂CH₃)₃⁺, NH(CH₃)₃⁺, ((CH₃CH₂CH₂CH₂)₄)P⁺ oder Kombinationen davon.

5. Die Zusammensetzung nach einem der Ansprüche 1, 2 oder 4, wobei Rf verzweigt oder cyclisch ist.

6. Die Zusammensetzung nach einem der vorstehenden Ansprüche, wobei der fluorierte Elastomerkautschuk mindestens eines umfasst von: (i) Copolymere, umfassend TFE- und eine Perfluoralkylvinylethermonomereinheiten; (ii) Copolymere, umfassend TFE- und eine Perfluoralkoxyvinylethermonomereinheiten; (iii) Copolymere, umfassend TFE- und Propylenmonomereinheiten; (iv) Copolymere, umfassend TFE-, Propylen- und VDF-Monomereinheiten; (v) Copolymere, umfassend VDF- und HFP-Monomereinheiten; (vi) Copolymere, umfassend TFE-, VDF- und HFP-Monomereinheiten; (vii) Copolymere, umfassend VDF- und Perfluoralkylvinylether-Monomereinheiten; (viii) Copolymere, umfassend CTFE- und VDF-Monomereinheiten; (ix) Copolymere, umfassend TFE- und VDF-Monomereinheiten; (x) Copolymere, umfassend TFE-, VDF- und Perfluoralkylvinylethermonomereinheiten; und (xi) Kombinationen davon.

7. Die Zusammensetzung nach einem der Ansprüche 1 bis 5, wobei der fluorierte Elastomerkautschuk ein Blockcopolymer ist, umfassend mindestens einen A-Block und mindestens einen B-Block.

8. Die Zusammensetzung nach einem der vorstehenden Ansprüche, wobei der fluorierte Elastomerkautschuk zu höchstens 5 Gew.-% die Härtungsstelle umfasst.

9. Die Zusammensetzung nach einem der vorstehenden Ansprüche, umfassend zu mindestens 3 bis höchstens 10 Gewichtsteilen das Co-Mittel pro 100 Gewichtsteile des fluorierten Elastomerkautschuks.

10. Die Zusammensetzung nach einem der vorstehenden Ansprüche, ferner umfassend ein Peroxid.

11. Die Zusammensetzung nach Anspruch 10, umfassend zu mindestens 0,25 Gewichtsteilen bis nicht mehr als 10 Gewichtsteilen das Peroxid pro 100 Gewichtsteile des fluorierten Elastomerkautschuks.

12. Die Zusammensetzung nach einem der vorstehenden Ansprüche, ferner umfassend einen Füllstoff, gegebenenfalls, wobei die Zusammensetzung zu weniger als 50 Gew.-% den Füllstoff, basierend auf dem Gewicht des fluorierten Elastomerkautschuks, umfasst.

13. Ein Gegenstand, umfassend die gehärtete Zusammensetzung nach einem der Ansprüche 1 bis 12.

14. Ein Verfahren zum Härten eines fluorierten Elastomerkautschuks, das Verfahren umfassend:
Erhalten eines fluorierten Elastomerkautschuks, umfassend Härtungsstellen, wobei die Härtungsstelle mindestens eines von Br, I oder Nitril umfasst;
Härten des fluorierten Elastomerkautschuks mit einem Peroxid und einem Co-Mittel, wobei das Co-Mittel der Formel I entspricht:
(CH₂=CHCH₂)₂-L¹-Rf-L²-(CH₂CH=CH₂)ₙ(H)₂₋ₙ,
wobei L¹ -NSO₂₋ oder -NCO- ist; L² -SO₂N- oder -CON- ist; n 1 oder 2 ist; und Rf eine zweiwertige perfluorierte Kohlenstoffgruppe ist, gegebenenfalls umfassend mindestens eine Etherbindung.

15. Die Zusammensetzung nach einem der Ansprüche 1 bis 12, wobei die Zusammensetzung zu weniger als 0,1 Gewichtsteilen eine nicht fluorierte, mehrfach ungesättigte Verbindung umfasst, die zwei oder mehr terminalen Ungesättigtheitsstellen pro 100 Gewichtsteile pro fluorierter Elastomerkautschuk aufweist.

## Revendications

1. Composition comprenant :
a. une gomme élastomère fluorée comprenant au moins 0,05 % en poids d'un site de durcissement, dans laquelle le site de durcissement comprend au moins un parmi brome, iode, nitrile ou des combinaisons de ceux-ci ;
b. un co-agent de Formule I ou un sel de celui-ci, dans laquelle la Formule I est
(CH₂=CHCH₂)₂-L¹-Rf-L²-(CH₂CH=CH₂)ₙ(H)₂₋ₙ
dans laquelle L¹ est -NSO₂- ou -NCO- ; L² est -SO₂N- ou -CON- ; n vaut 1 ou 2 ; et Rf est un groupe carboné perfluoré divalent, comprenant facultativement au moins une liaison éther.

2. Composition selon la revendication 1, dans laquelle le sel de Formule I comprend un sel formant un cation avec une valence de 1, 2 ou 3.

3. Composition selon l'une quelconque des revendications précédentes, dans laquelle le co-agent comprend au moins un parmi :
(CH₂=CHCH₂)₂NSO₂C₃F₆SO₂N(CH₂CH=CH₂)₂ ;
(CH₂=CHCH₂)₂NSO₂C₄F₈SO₂N(CH₂CH=CH₂)₂ ;
(CH₂=CHCH₂)₂NSO₂C₆F₁₀SO₂N(CH₂CH=CH₂)₂ ;
(CH₂=CHCH₂)₂NSO₂C₂F₄OC₂F₄SO₂N(CH₂CH=CH₂)₂ ;
(CH₂=CHCH₂)₂NCOC₄F₈CON(CH₂CH=CH₂)₂ ;
(CH₂=CHCH₂)₂NCOCF₂OC₂F₄OCF₂CON(CH₂CH=CH₂)₂ ;
(CH₂=CHCH₂)₂NCOCF₂OC₂F₄OC₂F₄OCF₂CON(CH₂CH=CH₂)₂ ;
(CH₂=CHCH₂)₂NCOC₃F₆SO₂N(CH₂CH=CH₂)₂ ;
(CH₂=CHCH₂NHSO₂C₃F₆CON(CH₂CH=CH₂)₂
et sels de celui-ci ; et
(CH₂=CHCH₂)₂NSO₂C₃F₆CONHCH₂CH=CH₂
et sels de celui-ci

4. Composition selon l'une quelconque des revendications précédentes, dans laquelle le sel est choisi parmi au moins un parmi : NH₄⁺, H₈O⁺, Na⁺, Li⁺, Cs⁺, Ca⁺², K⁺, Mg⁺², Zn⁺², et Cu⁺², N(CH₃)₄⁺, NH₂(CH₃)₂⁺, N(CH₂CH₃)₄⁺, NH(CH₂CH₃)₃⁺, NH(CH₃)₃⁺, ((CH₃CH₂CH₂CH₂)₄)P⁺ ou des combinaisons de ceux-ci.

5. Composition selon l'une quelconque des revendications 1, 2 ou 4, dans laquelle Rf est ramifié ou cyclique.

6. Composition selon l'une quelconque des revendications précédentes, dans laquelle la gomme élastomère fluorée comprend au moins un parmi : (i) des copolymères comprenant des motifs monomères de TFE et d'un éther perfluoroalkyl-vinylique ; (ii) des copolymères comprenant des motifs monomères de TFE et d'un éther perfluoroalcoxy-vinylique ; (iii) des copolymères comprenant des motifs monomères de TFE et de propylène ; (iv) des copolymères comprenant des motifs monomères de TFE, de propylène et de VDF ; (v) des copolymères comprenant des motifs monomères de VDF et de HFP ; (vi) des copolymères comprenant des motifs monomères de TFE, de VDF et de HFP ; (vii) des copolymères comprenant des motifs monomères de VDF et d'éther perfluoroalkyl-vinylique ; (viii) des copolymères comprenant des motifs monomères de CTFE et de VDF ; (ix) des copolymères comprenant des motifs monomères de TFE et de VDF ; (x) des copolymères comprenant des motifs monomères de TFE, de VDF et d'éther perfluoroalkyl-vinylique ; et (xi) des combinaisons de ceux-ci.

7. Composition selon l'une quelconque des revendications 1 à 5, dans laquelle la gomme élastomère fluorée est un copolymère séquencé comprenant au moins une séquence A et au moins une séquence B.

8. Composition selon l'une quelconque des revendications précédentes, dans laquelle la gomme élastomère fluorée comprend au plus 5 % en poids du site de durcissement.

9. Composition selon l'une quelconque des revendications précédentes, comprenant au moins 3 jusqu'à au plus 10 parties en poids du co-agent pour 100 parties en poids de la gomme élastomère fluorée.

10. Composition selon l'une quelconque des revendications précédentes, comprenant en outre un peroxyde.

11. Composition selon la revendication 10, comprenant au moins 0,25 partie en poids jusqu'à pas plus de 10 parties en poids du peroxyde pour 100 parties en poids de la gomme élastomère fluorée.

12. Composition selon l'une quelconque des revendications précédentes comprenant en outre une charge, facultativement, dans laquelle la composition comprend moins de 50 % en poids de la charge sur la base du poids de la gomme élastomère fluorée.

13. Article comprenant la composition durcie selon l'une quelconque des revendications 1 à 12.

14. Procédé de durcissement d'une gomme élastomère fluorée, le procédé comprenant :
l'obtention d'une gomme élastomère fluorée comprenant des sites de durcissement, dans lequel le site de durcissement comprend au moins un parmi Br, I ou nitrile ;
le durcissement de la gomme élastomère fluorée avec un peroxyde et un co-agent, dans lequel le co-agent est selon la Formule I :
(CH₂=CHCH₂)₂-L¹-Rf-L²-(CH₂CH=CH₂)ₙ(H)₂₋ₙ
dans lequel L¹ est -NSO₂- ou -NCO- ; L² est -SO₂N- ou -CON- ; n vaut 1 ou 2 ; et Rf est un groupe carboné perfluoré divalent, comprenant facultativement au moins une liaison éther.

15. Composition selon l'une quelconque des revendications 1 à 12, dans laquelle la composition comprend moins de 0,1 partie en poids d'un composé poly-insaturé non fluoré ayant deux sites d'insaturation terminaux ou plus pour 100 parties en poids par gomme élastomère fluorée.
